Europäisches Patentamt

European Patent Office

Office européen des brevets

(11) Veröffentlichungsnummer: **0 268 995**

A2

(12) **EUROPÄISCHE PATENTANMELDUNG**

(21) Anmeldenummer: 87117058.5

(22) Anmeldetag: 19.11.87

(51) Int. Cl.4: **C07K 15/14** , G01N 33/68 , A61K 37/02 , C07K 3/18 , //C12P21/00

(30) Priorität: 22.11.86 DE 3639920

(43) Veröffentlichungstag der Anmeldung: 01.06.88 Patentblatt 88/22

(84) Benannte Vertragsstaaten: AT BE CH DE ES FR GB GR IT LI LU NL SE

(71) Anmelder: HOECHST AKTIENGESELLSCHAFT Postfach 80 03 20 D-6230 Frankfurt am Main 80(DE)

(72) Erfinder: Hünig, Thomas, Dr. Seitzstrasse 4a D-8033 Planegg(DE) Erfinder: Tiefenthaler, Georg Parkstrasse 2 D-8000 München 2(DE) Erfinder: Mitnacht, Rita Gotthardstrasse 87 D-8000 München 21(DE) Erfinder: Meuer, Stefan, Dr. Am Wegweiser 5 D-6508 Alzey(DE)

(54) Homogenes Schaf-T11TS als Zwischenprodukt zur Herstellung von menschlichem T11TS und dessen Verwendung.

(57) Die Zielstruktur T11TS auf Schaf-Erythrocyten für die T11-Struktur auf menschlichen T-Zellen wird homogen gereinigt. Mit Hilfe des homogenen Schaf-T11TS kann ein anti T11TS-Antiserum mit Antikörpern hergestellt werden, die sowohl mit Schaf-T11TS wie auch mit T11TS auf menschlichen Erythrocyten reagieren. Schaf-T11TS sowie menschliches T11TS sind in Diagnostik und Therapie einzusetzen.

EP 0 268 995 A2

## Homogenes Schaf-T11TS als Zwischenprodukt zur Herstellung von menschlichem T11TS und dessen Verwendung.

Die spezifischen Abwehrzellen des Immunsystems, die Lymphocyten, werden bei der Abwehr pathogener Mikroorganismen oder maligner körpereigener Zellen aus einem ruhenden in einen aktivierten Zustand versetzt. In diesem aktivierten Zustand vermehren sie sich durch Zellteilung und reifen zu Effektorzellen, die z.B. virusinfizierte Zellen zerstören können.

Der Spezität immunologischer Reaktionen liegt das Prinzip der Klonselektion zugrunde. Verschiedene Lymphocytenklone tragen Rezeptoren für verschiedene körperfremde Strukturen, die Antigene. Bei Infektion z.B. mit Influenza Virus werden andere Lymphocytenklone aktiviert als bei Infektion mit Poliovirus.

Zusätzlich zu den Antigen-(z.B. Virus-) spezifischen Rezeptoren exprimieren Lymphocyten Oberflächenrezeptoren für regulatorische (stimulierende oder dämpfende) Signale, durch die das Ausmaß einer Immunreaktion entscheidend beeinflußt wird. Diese Rezeptoren können entweder hormonartige Botenstoffe , die Interleukine, binden, oder komplementäre Liganden auf anderen Zellen erkennen. Im letzteren Fall spricht man von Zellinteraktionsmolekülen.

Menschliche Thymus-abgeleitete (T-)Lymphocyten exprimieren ein Zelloberflächenmolekül, das traditionell als der Erythrocyten (E-)Rezeptor bekannt ist, nach neuerer Nomenklatur auch als T11 oder CD2 Antigen bezeichnet wird. Im klassischen Nachweisverfahren für menschliche T-Lymphozyten bilden Schaferythrocyten durch Bindung an den E-Rezeptor spontan Aggregate, sogenannte E-Rosetten, mit den T-Zellen. Der Ligand auf den Erythrocyten wird T11 Target Structure (T 11 Zielstruktur), kurz T11TS genannt.

Der E-Rezeptor ist mit Hilfe monoklonaler Antikörper biochemisch und funktionell charakterisiert worden. Interessanter Weise können manche dieser Antikörper gegen T11 die Aktivierung ruhender T-Lymphocyten verhindern, andere können hingegen T-Lymphocyten unter Umgehung des Antigen-spezifischen Rezeptors zur Zellteilung aktivieren [Howard, F.D., et al. J. Immunol. 126:2117 (1981). Kamoun, M.P., et al. J. Exp. Med. 153:207 (1981). Palacios, R., and Martinez-Maza, O.J. Immunol. 129:2497 (1982). Krensky, A.M., et al. J. Immunol. 131:611 (1983). Meuer, S.C., et al., Cell 36:897 (1984)].

Es gelang ferner, einen monoklonalen Antikörper gegen Schaferythrocyten zu selektionieren, der ihre Bindung an den E-Rezeptor vollständig blockiert und damit mit der gesuchten T11TS Struktur reagiert.

Mit Hilfe dieser monoklonalen Antikörper wurde Schaf-T11TS biochemisch als ein Glykoprotein von 42000 MG (Proteinanteil 30000) mit einem Isoelektrischen Punkt von 4.5 charakterisiert. Die Identifizierung des Schaf-T11TS Moleküls mit Hilfe eines monoklonalen Antikörpers, die Expression auf Erythrocyten und Leukocyten und die aufgeführten biochemischen Charakteristika wurden bereits publiziert [Hünig, T., J.Exp.Med. 162:890 (1985). Hünig, T., J.Immunol. 136:2103 (1986)].

Trotz der großen Schwierigkeiten, die die Reinigung eines Membranproteins bereitet, ist es gelungen Schaf-T11TS zur Homogenität zu reinigen. Überraschenderweise konnte mit Hilfe dieses homogenen Schaf-T11TS ein anti-T11TS Antiserum hergestellt werden, das mit menschlichem T11TS reagiert.

Schaf-und menschliches T11TS sowie ihre Varianten und monoklonale Antikörper gegen T11TS können Anwendung in Diagnostik und Therapie finden.

Die Erfindung betrifft somit:

1. Homogenes Schaf-T11TS mit
   a)einer Aminosäurezusammensetzung von

|        | mol%            |
|--------|-----------------|
| Asx    | 9,6             |
| Thr    | 5,9             |
| Ser    | 9,5             |
| Glx    | 14,1            |
| Pro    | 3,5             |
| Gly    | 7,9             |
| Ala    | 7,5             |
| Val    | 7,2             |
| Met    | 1,2             |
| Ile    | 5,5             |
| Leu    | 9,3             |
| Tyr    | 2,6             |
| Phe    | 4,6             |
| Lys    | 4,6             |
| His    | 2,6             |
| Arg    | 4,6             |
| Trp    | nicht bestimmt  |
| Cys    | nicht bestimmt  |

und

b) einem N-Terminus von
Val/Phe-Ser-Gln/Ser-Asp-Ile-Tyr-Gly-Ala-Met-Asn-Gly-Y-Val-Thr-Phe-Tyr-Val-Ser-Glu-Ser-Gln-Pro-Phe-Thr-Glu-Ile-Met-X-Lys
in dem X und Y eine natürlich vorkommende Aminosäure bedeuten, wobei Y vorzugsweise Serin ist, sowie dessen Varianten

2. Ein Verfahren zur immunologischen Bestimmung von menschlichem T11TS, das dadurch gekennzeichnet ist, daß

a) ein Tier einer geeigneten Spezies mit homogenem Schaf-T11TS immunisiert wird und

b) Antikörper aus dem Antiserum mit menschlichem T11TS zur Reaktion gebracht werden.

3. Menschliches T11TS-Glykoprotein mit 60.000 MG sowie dessen Varianten erhältlich durch das Verfahren wie unter 2) definiert.

4. Die Verwendung des menschlichen und/oder Schaf-T11TS wie unter 1) und 3) charakterisiert sowie dessen Varianten als Immunmodulator sowie zur Herstellung monoklonaler Antikörper.

Im folgenden wird die Erfindung detailliert, insbesondere in ihren bevorzugten Ausführungsformen, erläutert, sowie in den Patentansprüchen definiert.

Unter Varianten von T11TS versteht man Veränderungen am Molekül die durch Punkt-Deletion, Punkt-Mutation, beispielsweise Entfernung der Glykosylierungsstelle, Verkürzungen, z.B. Deletion des Transmembranteils, oder Verlängerungen erreicht werden, und die die biologische Aktivität des Moleküls als Suppressor oder Aktivator verbessern.

Mit Hilfe von monoklonalen Antikörpern gegen Schaf-T11TS, die nach an sich bekannten Methoden hergestellt werden können [Hünig T., J.Exp.Med. 162 , 890 (1985], wird das Molekül aus Detergenslysaten von Schaferythrocyten, bevorzugt unter Verwendung eines Octylphenolethylenoxidkondensats (NP-40) und in Anwesenheit eines Proteaseinhibitors, beispielsweise Phenylmethylsulfonylfluorid (PMSF), durch Immunaffinität stark angereichert. Durch präparative Gelelektrophorese, z.B. mit SDS-Polyacrylamid, bevorzugt unter Verwendung von radioaktiv markiertem Schaf-T11TS als "Tracer", und anschließende elektrophoretische Elution wird beispielsweise das Molekül zur Homogenität gereinigt. Die Aminosäurezusammensetzung kann nach Hydrolyse von Schaf-T11TS bestimmt werden. Nach Entfernung von Detergens und Salzresten durch geeignete Fällungsschritte können Schaf-T11TS bzw. einige durch tryptische Spaltung gewonnenen Fragmente partiell sequenziert werden.

Das homogen gereinigte Schaf-T11TS bietet die Möglichkeit ein Antiserum herzustellen mit dessen Hilfe menschliches T11TS, d.h. der entsprechende Ligand des Erythrocytenrezeptors menschlicher T-Zellen, nachgewiesen werden kann. Dazu wird ein Tier einer geeigneten Spezies, wie beispielsweise ein Kaninchen, mit homogenem Schaf T11TS unter Zugabe eines geeigneten Adjuvans (z.B. Freund's oder Aluminiumhydroxid) durch subcutane oder intramuskuläre Injektion immunisiert. Es können die in diesen Fällen üblichen Antigendosen angewendet werden. Die bevorzugte Dosis am Kaninchen ist 0,05 bis 1 mg pro Tier. In bestimmten Zeitabständen nach jeder Injektion wird dem Tier Blut abgenommen und auf Anwesenheit von Anti-T11TS Antikörper überprüft. Der Antikörpertiter kann durch Blockierung der Bindung von Schaferythrocyten an menschliche T-Lymphocyten verfolgt werden. Bei ausreichend hohem Titer werden größere Mengen Blut abgenommen und das Antiserum daraus gewonnen. Es ist auch möglich, die im Serum vorhandenen Antikörper vorher noch zu reinigen. Ein bevorzugte Methode dabei ist die Affinitätschromotographie.

Mit Hilfe von Radio-Immunpräzipitation aus Detergenslysaten von radioaktiv markierten Erythrocyten oder durch Immunoblot (Westernblot) kann nach der Reaktion des T11TS-Antigens mit den Antikörpern des Antiserums das Molekulargewicht des Antigens nachgewiesen werden. Die Markierung des Antigens kann in den für Proteinmarkierung bekannten Methoden erfolgen. Im Falle der radioaktiven Markierung wird als Radionuklid bevorzugt Jod 125 verwendet. Die Markierung kann dann nach der bekannten Chloramin T Methode (Int. Arch. Allergy 29, 185, 1966) erfolgen. Das anti Schaf-T11TS-Antiserum enthält überraschend auch Antikörper, die nicht nur Schaf-sondern auch menschliches T11TS erkennen können. Durch obengenannte biochemische Nachweisverfahren kann das T11TS-Molekül auf menschlichen Erythrocyten als ein Glykoprotein von etwa 60.000 MG bestimmt werden.

Mit Hilfe eines monoklonalen Antikörpers gegen Schaf-T11TS, der mit dem menschlichen T11TS kreuzreagiert, kann dieses aus Detergenslysaten von Erythrocyten aus menschlichem Gewebe durch Immunaffinität stark angereichert werden. Die Reinigung zur Homogenität kann entsprechend der Methode für das Schaf-T11TS durchgeführt werden.

Die Aminosäuresequenz von T11TS-Molekülen kann man mit Hilfe der c-DNA-Sequenzierung bestimmen. Dazu wird eine T11TS Protein-Mikrosequenzierung durchgeführt mit anschließender Übersetzung in die DNS-Sequenz. So erhaltene Oligonucleotidproben werden radioaktiv markiert mit Klonen inkubiert, die cDNA enthalten, die zur RNA aus T11TS haltigen Geweben komplementär ist.

Durch autoradiographische Verfahren werden die spezifisch hybridisierenden Klone identifiziert. Nach der Bestimmung der Nucleotidsequenz der T11TS c-DNA kann die Aminosäuresequenz von T11TS festgelegt werden.

Alternativ können die T11TS c-DNA Klone mit Hilfe des Antiserums identifiziert werden, wenn die c-DNA in einen Expressionsvektor kloniert wird.

Sowohl Schaf-wie auch menschliches T11TS kann als Immunmodulator eingesetzt werden, was durch folgende Beobachtung verdeutlicht wird: Außer über ihren Antigenspezifischen Rezeptor können menschliche T-Lymphozyten auch durch bestimmte Kombinationen von monoklonalen Antikörpern gegen den E-Rezeptor aktiviert werden. Es kann gezeigt werden, daß eines der Signale, die für die T-Zellen Aktivierung auf diesem "alternativen Weg" notwendig sind, durch die Bindung von T11TS und den E-Rezeptor gegeben wird.

In den aus der Literatur bekannten Experimenten wird der "alternative Weg" z.B. durch gleichzeitige Zugabe von zwei verschiedenen monoklonalen Antikörpern gegen verschiedene Teilbereiche ("Epitope") des E-Rezeptors zu Kulturen von T-Lymphozyten induziert. Der erfindungsgemäß überraschende Befund war, daß einer dieser monoklonalen Antikörper durch Schafserythrocyten ersetzt werden kann, d.h. daß Schafserythrocyten in Anwesenheit von nur einem anti-E-Rezeptor Antikörper die T-Lymphocyten aktivieren. Außerdem wird die durch die gleichzeitige Zugabe zweier monoklonaler Antikörper gegen den E-Rezeptor erreichte T-Zell Aktivierung durch Schafserythrocyten dramatisch gesteigert. Entscheidend für den Nachweis einer Beteiligung des T11TS-Moleküls ist der Befund, daß die stimulierende Aktivität von Schaferythrocyten vollständig durch den monoklonalen Antikörper gegen Schaf-T11TS neutralisiert wird. Die Bindung des T11TS-Moleküls an den E-Rezeptor menschlicher T-Lymphocyten gibt also ein aktivierendes Signal. Aus dieser Beobachtung ist daher zu schließen, daß Schaf-und menschliches T11TS sowie deren Varianten immunmodulatorische Eigenschaften besitzen. Je nach dem, ob diese Moleküle durch Bindung an den E-Rezeptor die T-Lymphozyten stimulieren, oder ob sie nur binden und damit die Bindung des im Körper natürlich vorkommenden T11TS an den E-Rezeptor verhindern, sind Stimulation oder Dämpfung des Immunsystems zu erwarten. Da bei zahlreichen Erkrankungen eine Modulation der immunologischen Reaktivität des Patienten angezeigt ist, besteht ein sehr breites potentielles Anwendungsgebiet für Pharmaka, die auf der Struktur von T11TS basieren.

Monoklonale Antikörper gegen T11TS und T11TS-spezifische Nukleinsäureproben können zur Bestimmung der T11TS-Expression in Geweben von Patienten eingesetzt werden und dadurch ein wichtiges Hilfsmittel für die Diagnose von Erkrankungen sein, an denen das Immunsystem entweder ursächlich (z. B. Autoimmunkrankheiten) oder indirekt (unzureichende Abwehrreaktion) beteiligt ist.

Darüberhinaus ist zu erwarten, daß monoklonale Antikörper gegen menschliches T11TS auch eine therapeutische Bedeutung im Sinne der oben dargelegten Immunmodulation haben.


Beispiele:

1. Reinigung und Darstellung von Schaf-T11TS.

a) Herstellung einer Immunaffinitäts-Säule.

Die Hybridoma-Zellinie, die einen monoklonalen Antikörper gegen Schaf T11TS produziert [Hünig T., J.Exp.Med. 162, 890 (1985)], wird intraperitoneal in Pristan-vorbehandelte Mäuse injiziert. Nach ca. 1 Woche wird die Aszites-Flüssigkeit aus der Bauchhöhle der Mäuse gewonnen. Sie enthält ca. 10 mg/ml des Antikörpers. Die Aszitesflüssigkeit wird gegen 20 mM TRIS-Puffer, pH 8,0, dialysiert und auf eine mit diesem Puffer äquilibrierte DEAE-Affigel-blue (BIORAD) Säule aufgetragen. Die Antikörper und die meisten anderen Proteine binden an die Säule. Bei Anlegen eines Kochsalzgradienten von 0-100 mM in TRIS, pH 8, eluiert der Antikörper selektiv bei etwa 70 mM NaCl. Er wird umdialysiert in Phosphat-gepufferte Kochsalz Lösung (Phosphate Buffer Saline, PBS), pH 7,2, und über Nacht mit Glutardialdehyd-aktivierten Glaskugeln (Boehringer Mannheim) rotiert (50 mg Antikörper auf 2 g Glas). Die Glaskugeln werden mit PBS gewaschen, bis kein Protein im Überstand nachweisbar ist, und in eine Säule (2,5 $\times$ 10 cm) mit 1 % NP-40 in PBS gefüllt.


b) Herstellung eines Schafserythrozyten Detergenslysats.

Frisches Schafsblut vom Schlachthof wird in Alsever's Lösung zur Vermeidung der Blutgerinnung aufgefangen. Die Blutzellen werden 2 mal in einem 5-fachen Volumen (Puffer : Blut) von Alsever's Lösung, dann einmal in PBS durch Zentrifugation bei 400 $\times$ g gewaschen. 1 Liter Zellsediment wird mit 1 Liter einer Lösung aus 1 % NP-40, 10 mM PMSF (Proteaseinhibitor) in PBS 30 min. auf Eis lysiert. Das Lysat wird 20 min. bei 22000 $\times$ g zentrifugiert und die nicht gelösten Zellbestandteile im Sediment verworfen. Der Überstand wird durch ein Glasfaserfilter (Sartorius) filtriert.


c) Anreicherung von T11TS durch Immunaffinität.

Das Erythrocytenlysat wird mit einer Geschwindigkeit von 1 ml/min. bei 4°C über die Immunaffinitäts-Säule gegeben. Die Glaskugeln werden dann zunächst mit 1 % NP-40 und 10 mM PMSF und 1-Liter PBS dann mit 0,5 M KCl im gleichen Puffer gewaschen. Zur Elution von Schaf-T11TS werden die Glaskugeln aus der Säule entfernt und in 3 ml einer Lösung aus 1 % NP-40 und 3.5 M NH₄SCN in PBS über Nacht rotiert. Der Überstand wird gegen 1 % NP-40 in PBS dialysiert, mit 10.000 cpm $^{125}$I markiertem T11TS versetzt, in Laemmli Gelpuffer aufgekocht und auf ein präparatives Laemmli Gel [(Laemmli, U.K. Nature (London) 227:680 (1970)] von 12 % Acrylamidgehalt aufgetragen. Nach Beendigung der elektrophoretischen Auftrennung wird das Gel eingefroren, und über Nacht an einen Röntgenfilm angelegt. Nach Identifizierung der T11TS-haltigen Bande im Gel anhand der Filmschwärzung wird diese ausgeschnitten, in das obere Ende eines Elektroelutionsapparates gelegt und nach Goding [(Goding, J.W., Annual Rev. Walter and Eliza Hall Inst. p.49 (1983-84)] über Nacht elektrophoretisch eluiert. Anhand der Radioaktivitätsverteilung wird die Vollständigkeit der Elution überprüft. Das gereinigte Produkt wird aus der Apparatur entnommen und durch SDS-Polyacrylamid-Gelektrophorese und Proteinfärbung auf seine Reinheit überprüft. Für die Immunisierung von Kaninchen wird das gereinigte T11TS gegen 0,5 % NP-40 in PBS umdialysiert. Für biochemische Analysen wie die Mikrosequenzierung wird es von Salz-und Detergensresten befreit. Dazu wird zunächst NP-40 zu einer Endkonzentration von 1 % zugesetzt. Durch Zugabe eines gleichen Volumens 20 % Trichloressigsäure in Wasser wird das NP-40 gemeinsam mit dem T11TS ausgefällt. Der Niederschlag wird 5 mal mit kaltem Aceton gewaschen und luftgetrocknet.

2. Identifizierung von menschlichem T11TS.

Ein Kaninchen wird 4 mal in 6 wöchigen Abständen mit je ca. 50 $\mu$g reinem T11TS immunisiert. Die erste Immunisierung erfolgt nach Adsorption des Proteins an Aluminiumhydroxid. Der T11TS-Lösung wird ein gleiches Volumen 20 % wäßrige Kali-Alaun-Lösung zugesetzt, das Hydroxid durch Zutröpfeln von 1 N NaOH ausgefällt, einmal in PBS gewaschen, in 2 ml PBS suspendiert und subkutan injiziert. Der Anstieg des Antikörpertiters wird durch Komplement-abhängige Lyse von Schafserythrozyten bei verschiedenen Kaninchenserum-Verdünnungen verfolgt. 1 Woche nach der 4. Immunisierung wird eine größere Menge Blut abgenommen und das Serum daraus gewonnen. Die Anwesenheit von Antikörpern gegen T11TS wird im Westernblot nachgewiesen: Membranen von Schafs-und Human-Erythrozyten werden in Laemmli-SDS-Polyacrylamid Gelen von 12 % Acrylamidgehalt aufgetrennt und elektrophoretisch auf Nitrozellulosemembranen transferiert. Nichtbesetzte Proteinbindungsstellen auf der Nitrozellulose werden durch Kälberserum abgesättigt. Die Membranen werden dann in einer Verdünnung des Antiserums inkubiert. Nachdem nichtgebundene Komponenten des Antiserums weggewaschen worden sind, werden die an das Antigen (T11TS) gebundenen Antikörper durch ein mit alkalischer Phosphatase gekoppeltes Ziege anti-Kaninchen Serum und ein chromogenes Substrat sichtbar gemacht. Schaf-T11TS zeigt eine breite Bande bei 42.000 MG, menschliches T11TS bei 60.000 MG.

3. Nachweis der Signalwirkung von T11TS.

Menschliche periphere T-Zellen werden aus frischem heparinisiertem Spenderblut durch Dichte-Zentrifugation über ®Ficoll-Paque, (Pharmacia, Polysaccharid 5,7 g, Diatrizoat 9 g, Edetate Calcium-Dinatrium Dichte 1,119) für 30 Min. bei 160 $^\times$ g und Filtration durch eine mit Nylonwolle gestopfte Spritze in BSS (balanced salt solution) mit 5 % fötalem Kälberserum stark angereichert. Die Zellen werden in Roswell-Park-Memorial-Institute Kulturmedium (RPMI 1640, GIBCO) mit 5 % fötalem Kälberserum, Penicillin/Streptomycin, nichtessentiellen Aminosäuren, und Natriumpyruvat bei einer Zelldichte von $10^5$ pro 0,2 ml Kulturvolumen in 96 Napf-Mikrotiterplatten mit rundem Boden ausgesät. In verschiedenen Ansätzen enthalten die Kulturen zusätzlich 1:300 verdünnte Aszitesflüssigkeit von Mäusen, die mit einer Hybridomzellinie angeimpft wurden, die einen monoklonalen Antikörper gegen das T11$_3$ Epitop des E-Rezeptors produziert oder $10^6$ Neuraminidasebehandelte Schafserythrocyten oder anti-T11$_3$ Antikörper und Schafserythrocyten. Die Zellen werden drei Tage in einem mit 6 % $CO_2$ in Luft begasten Brutschrank bei 37°C inkubiert und dann 16 Stunden mit 1$\mu$Ci $^3$H Thymidin weiterkultiviert. Die Zellen werden dann in einem Zellerntegerät (Automash, Dynatech) mit Wasser lysiert und über ein Glasfaserfilter gesaugt. Die makromolekulare DNS haftet am Filter. Der Einbau von radioaktiv markiertem Thymidin in die DNS dient als Maß für die Zellteilungsaktivität der T-Lymphozyten und wird nach Trocknen der Filter mit Hilfe einer Scintillationsflüssigkeit in einem $\beta$-Zähler bestimmt. T-Lymphozyten ohne Zusatz bauen typischer Weise ca. 300 counts pro Minute (cpm) ein, ebenso Kulturen, die nur anti T11$_3$ oder nur Schafserythrozyten erhalten haben. Kulturen, die beide Zusätze erhielten, bauen mindestens 5000 cpm ein. Dieser stimulatorische Effekt wird vollständig aufgehoben, wenn diesem Ansatz zusätzlich 50 ng des in Beispiel 1 erwähnten anti-Schaf-T11TS monoklonalen Antikörpers zugesetzt werden. Die stimulierende Wirkung der Schafserythrocyten kann also auf das T11TS-Molekül zurückgeführt werden.

**4. Nachweis der Signalwirkung von isoliertem T11TS.**

Schaf TIITS wird, wie in Beispiel 1. beschrieben, durch Immunaffinität mit Hilfe des monoklonalen Antikörpers aus Detergens-lysierten Schafserythrozyten gereinigt, jedoch nicht der denaturierenden SDS-Polyakrylamid-Gelektrophorese unterworfen. Das Präparat wird durch Dialyse weitgehend von Detergens befreit. Es heißt im folgenden "lösliches T11TS".

In dem in Beispiel 3. beschriebenen Kultursystem werden menschliche T-Lymphozyten ausgesät und mit folgenden Zusätzen stimuliert:

Ansatz 1: kein Zusatz.

Ansatz 2: Monoklonale Antikörper gegen die $T11_{2+3}$Epitope (Meuer, S.C. et al., 1984, Cell, Bd. 36, S. 897) in Form einer 1:5000 verdünnten Aszitesflüssigkeit (bei dieser Verdünnung sind die Antikörper, auch wenn sie beide zugesetzt werden, nicht stimulatorisch).

Ansatz 3: 1 μg lösliches T11TS.

Ansatz 4: Anti-$T11_{2+3}$ Antikörper 1:5000 und 1 μg lösliches T11TS.

Ansatz 5: Wie Ansatz 4, doch zusätzlich mit 5 μg des monoklonalen Antikörpers L180/1 (Hünig, T., J. Exp. Med. 162:890 (1985); Hünig, T., 3. Immunol. 136:2103 (1986)) gegen T11TS.

Die Aktivierung der T-Lymphozyten wird, wie im Beispiel 3 beschrieben, durch den Einbau radioaktiven Thymidins gemessen.

Ergebnis: Ansätze 1, 2, 3 und 5 weniger als 1000 cpm, Ansatz 4 ca. 15000 cpm. Die gleichzeitige Zugabe der stark verdünnten anti $T11_{2+3}$ Antikörper und des löslichen T11TS führen also zu einer mindestens 15-fachen Steigerung der T-Lymphozyten Proliferation gegenüber dem unstimulierten Wert. Die Aufhebung dieses Effektes durch den monoklonalen Antikörper aus detergenslysierten Schafserythrozyten zeigt, daß die Stimulatorische Wirkung des zugesetzten Präparats auf die Anwesenheit von T11TS zurückzuführen ist.

## Ansprüche

1. Homogenes Schaf-T11TS mit
   a) einer Aminosäurezusammensetzung von

|     | mol% |
|-----|------|
| Asx | 9,6 |
| Thr | 5,9 |
| Ser | 9,5 |
| Glx | 14,1 |
| Pro | 3,5 |
| Gly | 7,9 |
| Ala | 7,5 |
| Val | 7,2 |
| Met | 1,2 |
| Ile | 5,5 |
| Leu | 9,3 |
| Tyr | 2,6 |
| Phe | 4,6 |
| Lys | 4,6 |
| His | 2,6 |
| Arg | 4,6 |
| Trp | nicht bestimmt |
| Cys | nicht bestimmt |

und

b) einem N-Terminus von

Val/Phe-Ser-Gln/Ser-Asp-Ile-Tyr-Gly-Ala-Met-Asn-Gly-Y-Val-Thr-Phe-Tyr-Val-Ser-Glu-Ser-Gln-Pro-Phe-Thr-Glu-Ile-Met-X-Lys

in dem X und Y eine natürlich vorkommende Aminosäure bedeuten, wobei Y vorzugsweise Serin ist, sowie dessen Varianten.

2. Ein spezifisches Antiserum mit Antikörpern gegen Schaf-T11TS und menschliches T11TS, erhältlich durch das Verfahren gemäß Anspruch 4 und 5.

3. Menschliches T11TS Glykoprotein mit 60.000 MG erhältlich durch das Verfahren gemäß Anspruch 6 oder 7.

4. Verfahren zur Herstellung eines spezifischen Antiserums mit Antikörpern gegen Schaf-T11TS und menschliches T11TS, dadurch gekennzeichnet, daß ein Tier mit homogenem Schaf-T11TS immunisiert wird.

5. Verfahren nach Anspruch 4, dadurch gekennzeichnet, daß ein Kaninchen immunisiert wird.

6. Verfahren zur immunologischen Bestimmung von menschlichem T11TS, dadurch gekennzeichnet, daß

a) ein Tier einer geeigneten Spezies mit homogenem Schaf-T11TS immunisiert wird und

b) Antikörper aus dem Antiserum mit menschlichem T11TS zur Reaktion gebracht werden.

7. Verfahren nach Anspruch 6, dadurch gekennzeichnet, daß ein Kaninchen immunisiert wird.

8. Verwendung des menschlichen und/oder Schaf-T11TS gemäß Anspruch 1 und 3 sowie dessen Varianten als Immunmodulator sowie zur Herstellung monoklonaler Antikörper.

9. Verfahren zur Herstellung von homogenem Schaf-T11TS gemäß Anspruch 1, dadurch gekennzeichnet, daß mit Hilfe von monoklonalen Antikörpern gegen Schaf-T11TS, Schaf-T11TS aus Detergenslysaten von Schaferythrozyten angereichert wird und gegebenenfalls durch Gelelektrophorese gereinigt wird.

Patentansprüche für folgende Vertragsstaaten: ES, GR

1. Verfahren zur Herstellung von homogenem Schaf-T11TS mit
a) einer Aminosäurezusammensetzung von

|     | mol%          |
|-----|---------------|
| Asx | 9,6           |
| Thr | 5,9           |
| Ser | 9,5           |
| Glx | 14,1          |
| Pro | 3,5           |
| Gly | 7,9           |
| Ala | 7,5           |
| Val | 7,2           |
| Met | 1,2           |
| Ile | 5,5           |
| Leu | 9,3           |
| Tyr | 2,6           |
| Phe | 4,6           |
| Lys | 4,6           |
| His | 2,6           |
| Arg | 4,6           |
| Trp | nicht bestimmt |
| Cys | nicht bestimmt |

und

b) einem N-Terminus von
Val/Phe-Ser-Gln/Ser-Asp-Ile-Tyr-Gly-Ala-Met-Asn-Gly-Y-Val-Thr-Phe-Tyr-Val-Ser-Glu-Ser-Gln-Pro-Phe-Thr-Glu-Ile-Met-X-Lys
in dem X und Y eine natürlich vorkommende Aminosäure bedeuten, wobei Y vorzugsweise Serin ist, dadurch gekennzeichnet, daß mit Hilfe von monoklonalen Antikörpern gegen Schaf-T11TS, Schaf T11TS aus Detergenslysaten von Schaferythrozyten angereichert wird und gegebenenfalls durch Gelelektrophorese gereinigt wird.

2. Verfahren zur Herstellung eines spezifischen Antiserums mit Antikörpern gegen Schaf-T11TS und menschliches T11TS, dadurch gekennzeichnet, daß ein Tier mit homogenem Schaf-T11TS immunisiert wird.

3. Verfahren nach Anspruch 2, dadurch gekennzeichnet, daß ein Kaninchen immunisiert wird.

4. Verfahren zur immunologischen Bestimmung von menschlichem T11TS, dadurch gekennzeichnet, daß

a) ein Tier einer geeigneten Spezies mit homogenem Schaf-T11TS immunisiert wird und

b) Antikörper aus dem Antiserum mit menschlichem T11TS zur Reaktion gebracht werden.

5. Verfahren nach Anspruch 4, dadurch gekennzeichnet, daß ein Kaninchen immunisiert wird.

6. Verwendung des menschlichen und/oder Schaf-T11TS, erhältlich gemäß Anspruch 1 oder 4 sowie dessen Varianten als Immunmodulator sowie zur Herstellung monoklonaler Antikörper.